# NEUE EUROPÄISCHE PATENTSCHRIFT

(11) **EP 1 576 972 B2**
(45) Veröffentlichungstag und Bekanntmachung des Hinweises auf die Entscheidung über den Einspruch: **02.05.2012**
(45) Hinweis auf die Patenterteilung: 24.09.2008
(21) Anmeldenummer: 05101833.1
(22) Anmeldetag: 09.03.2005
(51) Int. Cl.: A61M 1/16, A61M 1/34

(54) **Verfahren und Gerätekombination zur Gestattung von Bedienereingaben an einem medizinischen Gerät**
Method and device combination for validating user input on a medical apparatus
Méthode et ensemble d'appareillage de validation des données entrées par un utilisateur dans un appareil médical

(30) Priorität: 20.03.2004 DE 102004013814
(43) Veröffentlichungstag der Anmeldung: 21.09.2005
(73) Patentinhaber: B. Braun Avitum AG, 49215 Glandorf (DE)
(72) Erfinder: Niemetz, Günter, 34212 Melsungen (DE); Hollstein, Rolf, 36211 Alheim (DE); Möller, Dirk, 34326 Altmorschen (DE); Wenzel, Ulrich, 34212 Melsungen (DE); Bock, Gerhard, 36289 Friedewald (DE); Heitmeier, Rolf, 34225 Baunatal (DE)
(74) Vertreter: von Kreisler Selting Werner

(56) Entgegenhaltungen:
- EP-A1- 0 796 588
- EP-A2- 1 136 087
- WO-A-99/10029
- WO-A2-03/060805
- WO-A2-03/092769
- DE-A1- 10 116 650
- DE-U1- 20 113 789
- US-A- 5 739 508
- US-A1- 2002 038 392
- US-A1- 2003 140 928
- US-A1- 2004 010 425
- US-A1- 2004 019 464
- US-A1- 2004 039 257
- 'Infrarot Schnittstelle 4008 H/S', Bd. M312561, 02 November 2000, FRESENIUS MEDICAL CARE Seiten 1 - 36

## Beschreibung

Die Erfindung betrifft ein Verfahren zur Gestattung von Bedienereingaben an einem medizinischen Gerät, das Programme mit wählbaren Programmparametern durchführen kann, sowie ein medizinisches Gerät, mit dem ein derartiges Verfahren ausführbar ist.

Medizinische Geräte, an denen das erfindungsgemäße Verfahren ausführbar ist, sind insbesondere Blutbehandlungsgeräte für die extrakorporale Blutbehandlung, und speziell Dialysegeräte, wie sie in EP 0 623 357 A1 oder DE 197 42 633 C2 beschrieben sind. Derartige Geräte haben einen Computer mit Bildschirm sowie eine Eingabevorrichtung mit Tasten, Schaltern, Knöpfen oder einer Folientastatur. Die Eingabevorrichtung kann auch in den Bildschirm integriert sein, wenn dieser als Touchscreen ausgebildet ist. Die Eingabevorrichtung ermöglicht Anwendereingaben zur Auswahl von Behandlungsmoden, zum Einstellen von Betriebsparametern oder zur Alarmquittierung.

In US 2003/0212314 A1 ist ein medizinisches Gerät beschrieben, bei dem die Anwesenheit einer Person vor dem medizinischen Gerät durch einen Detektor festgestellt wird. Die Parameter des medizinischen Gerätes lassen sich nur verstellen, wenn eine Person vor dem Gerät anwesend ist. Auf diese Weise können unbeabsichtigte Verstellungen durch Umwelteinflüsse zu einem großen Teil verhindert werden.

Im Bereich der gerätegestützten medizinischen Behandlung wird durch das Bedienungspersonal durch Auswahl verschiedener Behandlungsprogramme, Behandlungsparameter oder Alarmquittierungsfunktionen eine auf den Patienten speziell abgestimmte Behandlung durchgeführt. Der Nachteil üblicher Bedieneinheiten, wie z. B. Touchscreens, ist, dass durch versehentliches Berühren verhältnismäßig leicht eine Änderung von Programmen oder Parametern bewirkt werden kann. Beispielsweise ist es bei der Reinigung der Eingabeeinrichtung mit einem Tuch oder Lappen nicht sicher auszuschließen, dass eine unerwünschte Berührung erfolgt. Insbesondere sind wegen der geringen Größe der Bedien- und Anzeigefelder bei Touchscreens Fehlbedienungen durch versehentlichen Kontakt nur schwer erkennbar, was im Falle wichtiger Parameter schwerwiegende Folgen für den behandelten Patienten haben kann.

Die üblichen Eingabesicherungen, wie Passwortabfragen (DE 2 146 074), Erkennungskarten mit Magnetstreifen (WO 87/02160) oder Barcode-Abtastsysteme (US 5,384,756) haben gravierende Nachteile für den Benutzer oder die Behandlung. Die Nachteile der Passwortabfrage sind die große Dauer der manuellen Passworteingabe, die Beschränkung auf Zahlencodes und die Möglichkeit der Fehleingabe des Passworts unter Stresssituationen. Magnetische Erkennungskarten unterliegen dem mechanischen Verschleiß des Magnetstreifens beim Lesen und der mechanischen Beanspruchung der Karte bei unvorsichtiger Handhabung. Optische Abtastsysteme für Barcodeleser haben ähnliche Nachteile wie magnetische Erkennungskarten. Zusätzlich fordern sie einen direkten Sichtkontakt zwischen Sender und Empfänger.

WO 87/02160 beschreibt einen Computer, der medizinische Akten von Patienten enthält. Hierbei ist ein Zugangssystem mit eingeschränktem Zugang für Personal unterschiedlicher Gruppierungen vorgesehen. Dieses System mit eingeschränktem Zugang bezieht sich lediglich auf den Abruf von Daten aus der elektronischen Akte.

Ein Verfahren, das im wesentlichen dem Oberbegriff des Patentanspruches 1 entspricht, ist beschrieben in US 2002/0038392 A1. Dieses Verfahren sieht die Zuordnung von Identifizierungsvorrichtungen zum Arzt und zum Patienten vor. Die Identifizierungsvorrichten sind Transponder, die per Funkübertragung mit einem Controller kommunizieren, welcher ein medizinisches Infusionsgerät steuert. Dabei gibt es unterschiedliche Berechtigungsgrade, die die Person, welche die Identifizierungsvorrichtung trägt, zur Vornahme unterschiedlicher Handlungen ermächtigt. Zu dem Inhalt des Bediener-Transponders gehören unter anderem eine Patienten-information und Angaben über die IV-Medikation sowie Medikationsreportkomponenten.

In US 2004/0039257 A1 ist ein computergestütztes Infusionssystem beschrieben, das benutzerabhängige Zusatzberechtigungsgrade aufweist. Dabei wird zwischen medizinischem Pflegepersonal und technischem Servicepersonal unterschieden, wobei nur die Bediener-Identifikation des Servicepersonals die Änderung der Maschinenkonfiguration und von Serviceparametern ermöglicht.

Eine Gerätekonfiguration, die es nur entsprechend qualifiziertem Personal erlaubt, Alarme an einem medizinischen Gerät zu quittieren, ist beschrieben in WO 99/10029.

Der Erfindung liegt die Aufgabe zugrunde, ein Verfahren zur Gestattung von Bedienereingaben an einem medizinischen Gerät, das geeignet ist, eine medizinische Behandlung des Körpers eines Patienten nach vorbestimmten Programmen mit wählbaren Programmparametern durchzuführen, anzugeben.

Das erfindungsgemäße Verfahren ist durch den Patentanspruch 1 bezeichnet.

Die Erfindung bietet den Vorteil, dass die Zugangskontrolle mit der eindeutigen Bediener-Identifikation in einem mehrstufigen Hierarchiesystem erfolgen kann. Die Autorisierung zur Eingabe von Behandlungsparametern erfolgt in Abhängigkeit von der Bediener-Identifikation des jeweiligen Bedieners. Die Einteilung der Bediener-Identifikationen könnte in der Weise erfolgen, dass das pflegerische Personal einer Dialysestation die Zugriffsberechtigung für therapierelevante Parameter hat, wie Therapie starten oder stoppen und die Alarmquittierung. Sobald der Computer eine Identifizierung einer Person feststellt, die zum Servicepersonal gehört (Servicetechniker), erhält diese zusätzlich zu der Zugangsberechtigung des pflegerischen Personals die Berechtigung, Konfigurations- und Serviceparameter einzustellen. Die Erfindung erlaubt also unterschiedliche Zugangsberechtigungen zu unterschiedlichen Programmen bzw. Funktionen sowie unterschiedliche Berechtigungen, diese Programme bzw. Funktionen zu aktivieren oder zu verändern.

Bei verschiedenen Behandlungen, wie beispielsweise der Dialyse, werden für jede Behandlung manuell Therapieprotokolle geführt. Die Erfindung ermöglicht es, ein solches Protokoll automatisch zu erstellen. Das medizinische Gerät erstellt in seinem Datenspeicher eine Datenstruktur, die dem manuell geführten Protokoll entspricht. Es speichert alle Patientendaten, wie Name, Kennnummer, Gewicht und die eingestellten Therapiewerte, wie Behandlungszeit, Ultrafiltrat-Abnahmemenge, Leitfähigkeit der Dialysierflüssigkeit. Zusätzlich zu den abgespeicherten Daten wird die aktuelle Bediener-Identifikation gespeichert, die zum Zeitpunkt der Dateneingabe von einer Leseeinheit übermittelt wird. Auf diese Weise enthält das Protokoll Angaben über die Identität der Bedienungsperson, die die Einstellungen vorgenommen und die Behandlung überwacht hat.

Die Bediener-Identifikation ist in einem von dem Bediener mitgeführten Transponder enthalten, der berührungslos mit einer Leseeinheit des medizinischen Gerätes kommuniziert. Eine solche Transponder-Lösung hat den Vorteil der einfachen Handhabung und eines geringen Zeitaufwandes für die Identifikation. Ein Transponder ist ein Gerät, das berührungslos auf eine Abfrage durch die mit dem Computer verbundene Leseeinheit reagiert und als Antwort auf eine solche Abfrage ein digitales Signal liefert, welches die betreffende Person identifiziert. Transponder benötigen in der Regel keine eigene Energieversorgung. Ein Beispiel eines Transponders, der die Kompatibilität der Komponenten eines Medizingerätes sicherstellt, ist in DE 20113789 der Anmelderin beschrieben.

Durch Verwendung von Transpondern, die entsprechende Speicher enthalten, besteht auch die Möglichkeit, das jeweilige Protokoll durch die Leseeinheit, die dann als Schreib-Leseeinheit ausgeführt ist, in den Transponder einzulesen. So kann am Ende der Behandlung das Protokoll auf dem Transponder des Bedieners abgespeichert werden, um später einem anderen Rechnersystem, das ebenfalls mit einer Leseeinheit für den Transponder ausgerüstet ist, zugeführt zu werden. Hier folgt eine weitere Verarbeitung der Daten zu Dokumentations- und Qualitätssicherungszwecken. Gleiches kann auch erreicht werden, wenn das medizinische Gerät mittels Datenkommunikation einem anderen Rechensystem das Protokoll am Ende der Behandlung oder kontinuierlich während der Behandlung zusammen mit der Bediener-Identifikation überträgt. Das Protokoll kann auch am medizinischen Gerät direkt ausgedruckt werden.

Die Erfindung sieht vor, den Patienten jeweils eine Patienten-Identifikation zuzuordnen und jeden Patienten mit einem Transponder auszustatten, in dem diese Identifikation gespeichert ist. Die Patienten-Identifikation enthält auch eine Angabe darüber, dass es sich um einen Patienten (und nicht um eine Bediener-Person) handelt. Da der Transponder mit einem hinreichend großen Speicher versehen ist, wird das Behandlungsprotokoll in einen Patienten-Transponder eingelesen, einschließlich der Bediener-Identifikation. Auf diese Weise führt der Patient die Protokolle vorheriger Behandlungen im Transponder mit sich. In einem derartigen System besteht auch die Möglichkeit, auf den Transponder bereits die Behandlungsparameter für nachfolgende Therapien abzuspeichern. Diese Behandlungsdaten werden unmittelbar vor der nächstfolgenden Behandlung von der Leseeinheit des medizinischen Gerätes gelesen und in den Computer des medizinischen Gerätes übernommen. Die Bedienungsperson muss dann noch ihre Bediener-Identifikation eingeben, um die Behandlung zu starten.

Im folgenden wird unter Bezugnahme auf die Zeichnungen ein Ausführungsbeispiel der Erfindung näher erläutert, wobei diese Erläuterungen nicht dahingehend zu verstehen sind, dass sie den Schutzbereich der Erfindung einschränken. Dieser wird vielmehr durch die Patentansprüche bestimmt.

### Es zeigen:

Fig. 1 eine schematische Darstellung eines Netzwerks, an das mehrere medizinische Geräte angeschlossen sind, wobei jedes medizinische Gerät eine Leseeinheit für Transponder aufweist, und

Fig. 2 eine schematische Darstellung der Datenübertragung zwischen einem Transponder und der Leseeinheit.

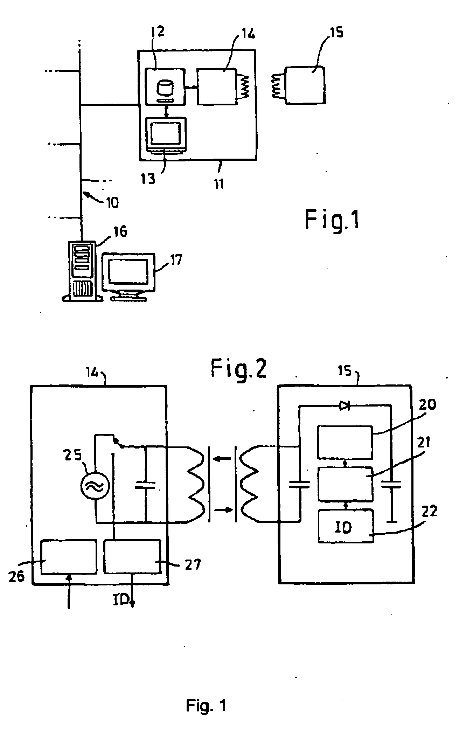

Fig. 1 zeigt eine Dialysestation, die ein Datennetz 10 enthält, an das zahlreiche medizinische Geräte 11 angeschlossen sind, bei denen es sich hier um Dialysemaschinen handelt. Jedes medizinische Gerät 11 enthält einen Computer 12 mit Software und Datenspeicher sowie eine Ein-/Ausgabevorrichtung 13, bei der es sich hier um einen Bildschirm mit Touchscreen-Funktion handelt.

Mit dem Computer 12 ist eine Schreib/Leseeinheit 14 verbunden, die berührungslos mit Transpondern 15 kommunizieren kann. Die Transponder 15 enthalten ein Radio-Tag, welches über Funkwellen mit der Schreib/Leseeinheit 14 kommuniziert, und einen Datenspeicher zum Abspeichern empfangener Daten. Die Kommunikation zwischen Leseeinheit und Radio-Tag ist bidirektional.

An das Netzwerk 10 ist ferner ein Server 16 mit Bildschirm 17 angeschlossen. Der Server 16 enthält einen umfangreichen Datenspeicher, auf den die Computer 12 sämtlicher Geräte 11 zugreifen können. Der Server 16 kann seinerseits an ein übergeordnetes Netz angeschlossen sein, beispielsweise das Internet oder ein Intranet.

In Fig. 2 ist das Zusammenwirken von Leseeinheit 14 und Transponder 15 dargestellt. Beide kommunizieren über Radiowellen miteinander. Der Transponder 15 enthält eine Energiequelle 20, eine Steuerung 21 und einen Speicher 22, in welchem u. a. die Bediener-Identifikation ID des Besitzers des Transponders enthalten ist. Der Speicher 22 kann auch dazu benutzt werden, Protokolle abzuspeichern.

Die Schreib/Leseeinheit 14 enthält einen Oszillator 25 zur Erzeugung der Trägerfrequenz für die Datenübertragung sowie Schnittstellen 26 und 27 für die Kommunikation mit dem Computer 12.

Die Schreib/Leseeinheit 14 erkennt einen in ihrer Nähe befindlichen Transponder 15 und fragt dessen gespeicherte Identifikation ID. Daraufhin sendet der Transponder die Identifikation ID an die Leseeinheit. Die Identifikation ID enthält eine Angabe über die betreffende Person, nämlich, ob die Person dem Pflegepersonal oder dem technischen Servicepersonal angehört. In beiden Fällen ergeben sich unterschiedliche Zugangsberechtigungen zur Änderung bzw. Einstellung von Programmen und Programmparametern am Bildschirm 13.

Es gibt auch Transponder 15 für Patienten. Bei diesen enthält die Identifikation ID eine Angabe über den Patientenstatus des Besitzers. In dem Speicher 22 können weitere persönliche Daten des Besitzers gespeichert sein, wie Name, Kennnummer, Gewicht u. dgl. In den Speicher können Therapiewerte, wie Behandlungszeit, Ultrafiltrat-Abnahmemenge, Leitfähigkeit usw. eingespeichert werden. Am Ende der Behandlung kann das Protokoll, das der Computer 12 von der Behandlung angefertigt hat, in den Transponder 15 der Bedienungsperson und/oder den Transponder 15 des Patienten rückgespeichert werden. Auf diese Weise besteht die Möglichkeit, das Behandlungsprotokoll in einen nicht an das Datennetz 10 angeschlossenen Computer zu übertragen.

Im Computer 12 wird anhand der Statusangabe, die einer Identifikation ID zugeordnet ist, die Zugangsberechtigung zur Einstellung bestimmter Programme oder Programmparameter festgelegt. Die Statusangabe besagt, ob die betreffende Person dem Pflegepersonal, dem technischen Servicepersonal oder einer anderen Gruppe angehört. Die Statusangabe kann Bestandteil der Identifikation ID sein. Andererseits besteht auch die Möglichkeit, dass anhand einer im Computer 12 oder im Server 16 gespeicherten Liste anhand der Identifikation ID festgestellt wird, welcher Status der betreffenden Person zugeordnet ist. Hier besteht auch die Möglichkeit zu überprüfen, ob die Person für das betreffende medizinische Gerät zugelassen ist. Hierbei wird eine personenbezogene Berechtigung im Computersystem hinterlegt.

In der nachstehenden Tabelle 1 ist ein Beispiel für verschiedene Zugriffsebenen dargestellt, die jeweils einem Status eines möglichen Bedieners zugeordnet sind. Die "Einstellfreigaben" geben diejenigen Einstellungen an, zu denen der zugeordnete Bediener autorisiert ist. Wenn das Gerät den Status des Bedieners festgestellt hat, ermöglicht es automatisch die entsprechenden Einstellfreigaben.

**Tabelle 1**

| **Zugriffsebene** | **Einstellfreigaben** | **Status** |
|---|---|---|
| 0 | keine, nur Istwerte anzeigen | Patient |
| 1 | Bedieneingaben und Alarmquittierung | Pflegepersonal |
| 2 | Behandlungsgrenzwerte, Bedieneingaben und Alarmquittierung | Arzt |
| 3 | Maschinenkonfiguration, Serviceparameter, Behandlungsgrenzwerte, Bedieneingaben und Alarmquittierung | technisches Servicepersonal |

Der Ausdruck "Maschinenkonfiguration" beinhaltet Angaben über Zusatzgeräte und Zusatzfähigkeiten der Maschine, wie z. B. Single-Needle-Ausstattung. "Serviceparameter" sind Kalibrier-werte und maschinentechnische Grenzwerte.

## Patentansprüche

1. Verfahren zur Gestattung von Bedienereingaben an einem medizinischen Gerät (11), das geeignet ist, eine medizinische Behandlung des Körpers eines Patienten nach vorbestimmten Programmen mit wählbaren Programmparametern durchzuführen, bei welchem
eine Bediener-Identifikation (ID) in einen das medizinische Gerät (11) steuernden Computer (12) eingegeben wird,
in Abhängigkeit von der Bediener-Identifikation unterschiedliche Zugangsberechtigungen zum Auswählen von Programmen und/oder zur Änderung von Programmparametern und/oder zur Eingabe von Alarmquittierungen erteilt werden,
in dem Computer (12) Patientendaten gespeichert werden, denen jeweils die ausgewählten Programme und Programmparameter zugeordnet werden und denen ferner die Bediener-Identifikation des verantwortlichen Bedieners hinzugefügt wird, wodurch ein Protokoll über einen Behandlungsablauf erstellt und gespeichert wird, und
die Bediener-Identifikation (ID) in einem von dem Bediener mitgeführten Transponder (15) enthalten ist, der berührungslos mit einer Leseeinheit (14) des medizinischen Gerätes (11) kommuniziert,
**dadurch gekennzeichnet,**
**dass** das Protokoll über den Behandlungsablauf und die Bediener-Identifikation auf einen Patienten-Transponder übertragen und dort abgespeichert werden.

2. Verfahren nach Anspruch 1, wobei zwischen medizinischem Pflegepersonal und technischem Servicepersonal unterschieden wird und wobei nur die Bediener-Identifikation des Servicepersonals die Änderung der Maschinenkonfiguration und von Serviceparametern ermöglicht.

3. Verfahren nach Anspruch 1 oder 2, bei welchem das medizinische Gerät Sensoren aufweist, deren Messdaten in dem Protokoll über den Behandlungsablauf aufgezeichnet werden.

4. Verfahren nach einem der vorhergehenden Ansprüche, **dadurch gekennzeichnet, dass** das auf dem Transponder abgespeicherte Protokoll für eine weitere Verarbeitung zu Dokumentations- und Qualitätssicherungszwecken einem anderen Rechnersystem zugeführt wird.

5. Gerätekombination aus einem medizinischen Gerät, insbesondere Blutbehandlungsgerät, für die extrakorporale Blutbehandlung, einem Bediener-Transponder (15) und einem Patienten-Transponder, mit einem die Blutbehandlung steuernden Computer (12), der Programme mit wählbaren Programmparametern durchführen kann, wobei die Zugangsberechtigung zur Auswahl von Programmen, Programmparametern und Alarmquittierungen in Abhängigkeit von einer verschiedenen Bedienern individuell zugeordneten Bediener-Identifikation (ID) bestimmt wird,
wobei die Bediener-Identifikation (ID) in dem Bediener-Transponder (15) enthalten ist und eine Patienten-Identifikation in einem Patienten Transponder enthalten ist, wobei die Transponder mit einer Leseeinheit (14) des medizinischen Gerätes (11) korrespondieren,
und wobei die Leseeinheit (14) eine Schreib-Leseeinheit ist und jeder Transponder einen Speicher (22) aufweist, der Daten enthält, die er von der Schreib-Leseeinheit empfangen hat,
**dadurch gekennzeichnet, dass** die in dem Speicher des Patienten-Transponders enthaltenen Daten ein Protokoll über den Behandlungsablauf und die Bediener-Identifikation umfassen.

## Claims

1. Method for allowing operator entries at a medical instrument (11) which is suitable for carrying out a medical treatment of the body of a patient according to predetermined programs using selectable program parameters, wherein
an operator identification (ID) is entered into a computer (12) controlling said medical instrument (11);
in dependence on the operator identification, different access authorizations for selecting programs and/or modifying program parameters and/or entering alarm acknowledgements are granted;
patient data are stored in the computer (12), to which patient data the selected programs and program parameters are assigned and further the operator identification of the responsible operator is added, whereby a record on the treatment process is prepared and stored, and
the operator identification (ID) is contained in a transponder (15) carried along by the operator, said transponder contactlessly communicating with a read unit (14) of the medical instrument (11),
**characterized in that**
the record on the treatment process and the operator identification are transmitted to a patient transponder and stored there.

2. Method according to claim 1, wherein a distinction is made between the medical nursing personnel and technical service personnel, and wherein only the operator identification of the service personnel allows for modification of the machine configuration and service parameters.

3. Method according to claim 1 or 2, wherein the medical instrument comprises sensors whose measuring data are registered in the record on the treatment process.

4. Method according to one of the preceding claims, wherein the record stored in the transponder is supplied to another computer system for further treatment for the purpose of documentation and quality assurance.

5. Combination of instruments comprising a medical instrument, in particular blood treatment instrument, for extracorporeal blood treatment, and a transponder (15), comprising a computer (12) controlling the blood treatment, said computer being capable of executing programs with selectable program parameters, wherein the access authorization with regard to selection of programs, program parameters and alarm acknowledgements is determined in dependence on an operator identification (ID) individually assigned to different operators, wherein
the operator identification (ID) or a patient identification is contained in a transponder (15) which communicates with a read unit (14) of the medical instrument (11),
the read unit (14) is a read/write unit and the transponder (15) comprises a memory (22) for storing data received from the read unit (14),
**characterized in that**
these data comprise a record on the treatment process and information about the identity of the patient or the operator identification, respectively.

## Revendications

1. Procédé pour permettre des entrées par un opérateur dans un appareil médical (11) apte à effectuer un traitement médical du corps d'un patient selon des programmes prédéterminés avec des paramètres de programme éligibles, dans lequel
un code d'identification de l'opérateur (ID) est entré dans un ordinateur (12) commandant l'appareil médical (11),
en fonction du code d'identification de l'opérateur, différentes autorisations d'accès sont accordées pour sélectionner des programmes et/ou modifier des paramètres de programme et/ou entrer des acquittements d'alarme,
des données des patients son stockées dans l'ordinateur (12), auxquelles les programmes et paramètres de programmes choisis sont respectivement associés, et auxquelles, en outre, le code d'identification de l'opérateur de l'opérateur responsable est ajouté, dont un protocole de cours d'un traitement est établi et stocké, et
le code d'identification de l'opérateur (ID) est contenu dans un transpondeur (15) emmené par l'opérateur, qui communique de manière sans contact avec un dispositif de lecture (14) de l'appareil médical (11),
**caractérisé en ce que**
le protocole de cours du traitement et le code d'identification de l'opérateur sont transmis à un transpondeur patient et sont stockés sur le même.

2. Procédé selon la revendication 1, dans lequel est distingué le personnel sanitaire médical du personnel de service technique, et dans lequel seulement le code d'identification de l'opérateur du personnel de service permet la modification de la configuration de la machine et des paramètres de service.

3. Procédé selon la revendication 1 ou 2, dans lequel l'appareil médical comprend des capteurs dont les données de mesure sont notées dans le protocole de cours du traitement.

4. Procédé selon une des revendications précédentes, **caractérisé en ce que** le protocole stocké dans le transpondeur est transmit à un autre système ordinateur pour le traitement ultérieur à la fin de documentation et de l'assurance de la qualité.

5. Combinaison d'appareils formée par un appareil médical, particulièrement un appareil de traitement du sang, pour le traitement extracorporel, et un transpondeur (15), comprenant un ordinateur (12) commandant le traitement du sang et apte à exécuter des programmes avec paramètres éligibles, l'autorisation d'accès pour la sélection de programmes, de paramètres de programme et pour l'acquittement d'alarmes étant déterminée en fonction d'un code d'identification de l'opérateur (ID) associé individuellement à différents opérateurs, dans lequel
le code d'identification de l'opérateur (ID) ou un code d'identification f'un patient est contenu dans le transpondeur (15) communicant avec un dispositif de lecture (14) de l'appareil (11) médical,
le dispositif de lecture (14) est un dispositif de lecture et d'enregistrement et le transpondeur (15) comprend une mémoire (22) comprenant des données reçues à partir du dispositif de lecture et d'enregistrement,
**caractérisé en ce que**
ces données comprennent un protocole de cours du traitement et des informations sur l'identité du patient ou le code d'identification de l'opérateur, respectivement.
